# EUROPÄISCHE PATENTSCHRIFT

(11) **EP 0 584 653 B1**
(45) Veröffentlichungstag und Bekanntmachung des Hinweises auf die Patenterteilung: **18.09.1996**
(21) Anmeldenummer: 93112874.8
(22) Anmeldetag: 11.08.1993
(51) Int. Cl.: C07C 31/125, A01N 27/00, A01N 31/02

(54) **Schabenbekämpfungsverfahren und Mittel zur Bekämpfung von Schaben**
Method of controlling cockroaches and agents to control them
Procédé de lutte contre les blattes et compostions pour combattre les luttes

(30) Priorität: 24.08.1992 DE 4228002
(43) Veröffentlichungstag der Anmeldung: 02.03.1994
(73) Patentinhaber: BAYER AG, 51368 Leverkusen (DE)
(72) Erfinder: Scherkenbeck, Jürgen Dr., D-51381 Leverkusen (DE); Nentwig, Günther Dr., D-51061 Köln (DE); Lenz, Jürgen Georg Dr., D-51061 Köln (DE); Boeckh, Jürgen Prof.Dr., D-93152 Nittendorf (DE); Wendler, Gernot Prof. Dr., D-50374 Erftstadt (DE); Dambach, Martin Prof. Dr., D-51399 Burscheid (DE); Krüger, Bernd-Wieland, Dr., D-51467 Bergisch Gladbach (DE)

(56) Entgegenhaltungen:
- 'Chemical Abstracts10th Collective Chemical Substance Index' , COLUMBUS OHIO,US * Seite 37007 *
- 'Chemical Substance Index 10th Collective Substance Index' , COLUMBUS OHIO,US * Seite 63753 *
- TETRAHEDRON, (INCL. TETRAHEDRON REPORTS) Bd. 48, Nr. 15 , April 1992 , OXFORD GB Seiten 3139 - 3146 E HEDENSTRÖM ET AL
- CHEMICAL ABSTRACTS, vol. 118, no. 15, 12. April 1993, Columbus, Ohio, US; abstract no. 141790z, A TAI ET AL Seite 239 ;Spalte 2 ;
- CHEMICAL ABSTRACTS, vol. 107, no. 5, 3. August 1987, Columbus, Ohio, US; abstract no. 36894m, M AUGUSTYNOWICZ ET AL Seite 437 ;Spalte 1 ;
- CHEMICAL ABSTRACTS, vol. 105, no. 25, 22. Dezember 1986, Columbus, Ohio, US; abstract no. 223086v, K LOCKEY ET AL Seite 482 ;Spalte 1 ;
- CHEMICAL ABSTRACTS, vol. 76, no. 19, 8. Mai 1972, Columbus, Ohio, US; abstract no. 110556m, L JACKSON ET AL Seite 265 ;Spalte 1 ;
- CHEMICAL ABSTRACTS, vol. 72, no. 19, 11. Mai 1970, Columbus, Ohio, US; abstract no. 97733p, L JACKSON ET AL Seite 103 ;Spalte 1 ;
- CHEMICAL ABSTRACTS, vol. 72, no. 19, 11. Mai 1970, Columbus, Ohio, US; abstract no. 97732n, K TARTIVITA ET AL Seite 103 ;Spalte 1 ;
- DATABASE WPI Week 7912, Derwent Publications Ltd., London, GB; AN 79-23097B & JP-A-54 020 868 (EARTH SEIYAKU) 16. Februar 1979
- DATABASE WPI Week 7736, Derwent Publications Ltd., London, GB; AN 77-64270Y & JP-B-52 031 416 (YAMABUN YUKA KK) 15. August 1977
- DATABASE WPI Week 6800, Derwent Publications Ltd., London, GB; AN 67-06393H & JP-B-44 011 920 (SANKYO)

## Beschreibung

Die vorliegende Erfindung betrifft die neue Verwendung von bestimmten chemischen Verbindungen als Lockstoffe bei der Bekämpfung von Schaben, Schabenbekämpfungsmittel, die diese Verbindungen enthalten sowie neue Lockstoffe und ein Verfahren zu deren Herstellung.

Schabenbefall stellt in Haushalt und Gewerbe ein erhebliches hygienisches Problem dar, das die Bekämpfung der Schaben erforderlich macht. Aufgrund ihrer Lebensweise sind Schaben jedoch schwer bekämpfbar. Ein besonderes Problem liegt dabei darin, die Schaben an mechanischen Bekämpfungsvorrichtungen (Fallen) oder insektizid wirksamen Mitteln zu konzentrieren. Zu diesem Zwecke wurden bereits die Sexualpheromone der amerikanischen Schabe (Periplaneta americana) eingesetzt (vgl. Bell 1986, Pesticide Control N. 12), wobei deren Spezifität und Wirksamkeit nicht immer zu befriedigenden Ergebnissen führte.

### Es wurde nun gefunden, daß die Verbindungen der allgemeinen Formel I

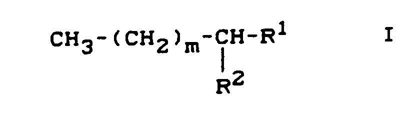

in welcher
- R¹: für C₁-C₅-Alkyl oder Hydroxy-C₁-C₅-alkyl steht;
- R²: für C₁-C₅-Alkyl steht; und
- m: für eine ganze Zahl von 10 bis 20 steht,
besonders vorteilhaft bei der Bekämpfung von Schaben eingesetzt werden können.

Die Verbindungen der allgemeinen Formel I weisen gegenüber Schaben anlockende Wirkungen auf und sind dazu geeignet, die Schaben an bestimmten Orten zu konzentrieren, wo sie mit mechanischen und/oder chemischen Mitteln bekämpft werden können. Die Verbindungen der Formel I sind hoch wirksam, stabil und durch Synthese leicht zugänglich. Die Verbindungen der allgemeinen Formel I können als Einzelverbindungen oder in Mischungen untereinander eingesetzt werden.

In der obigen allgemeinen Formel sowie in den unten aufgeführten allgemeinen Formeln und Restedefinitionen bedeuten:

Alkyl als solches oder als Bestandteil eines anderen Restes geradkettiges oder verzweigtes (vorzugsweise geradkettiges) Alkyl mit 1 bis 5, vorzugsweise 1 bis 4 und besonders bevorzugt 1 bis 3 Kohlenstoffatomen, wobei Methyl, Ethyl, n- und i-Propyl, n-, i-, s- und t-Butyl speziell genannt seien.
- R¹: steht vorzugsweise für C₁-C₄-Alkyl oder HydroxyC₁-C₄-alkyl.
- R²: steht vorzugsweise für C₁-C₃-Alkyl.
- m: steht vorzugsweise für eine ganze Zahl von 11 bis 19.

Erfindungsgemäß bevorzugt werden die Verbindungen der allgemeinen Formel I verwendet, in welchen eine Kombination dieser vorstehend als vorzugsweise aufgeführten Bedeutungen vorliegt.
- R¹: steht besonders bevorzugt für C₁-C₃-Alkyl oder Hydroxy-C₁-C₃-alkyl (vorzugsweise 1-Hydroxy-C₁-C₃-alkyl),
- R²: steht besonders bevorzugt für Methyl oder Ethyl.
- m: steht besonders bevorzugt für eine ganze Zahl von 12 bis 18.

Erfindungsgemäß besonders bevorzugt werden die Verbindungen der allgemeinen Formel I verwendet, in welchen eine Kombination dieser vorstehend als besonders bevorzugt aufgeführten Bedeutungen vorliegt.
- R¹: steht ganz besonders bevorzugt für Methyl, Ethyl (vorzugsweise Ethyl) oder Hydroxyethyl (vorzugsweise 1-Hydroxyethyl).
- R²: steht ganz besonders bevorzugt für Methyl.
- m: steht ganz besonders bevorzugt für eine ganze Zahl von 13 bis 17.

Erfindungsgemäß ganz besonders bevorzugt werden die Verbindungen der allgemeinen Formel I verwendet, in welchen eine Kombination dieser vorstehend als ganz besonders bevorzugt aufgeführten Bedeutungen vorliegt.

Beispiele für Verbindungen der Formel I, welche besonders vorteilhaft erfindungsgemäß verwendet werden können, sind aus der folgenden Tabelle 1 ersichtlich:

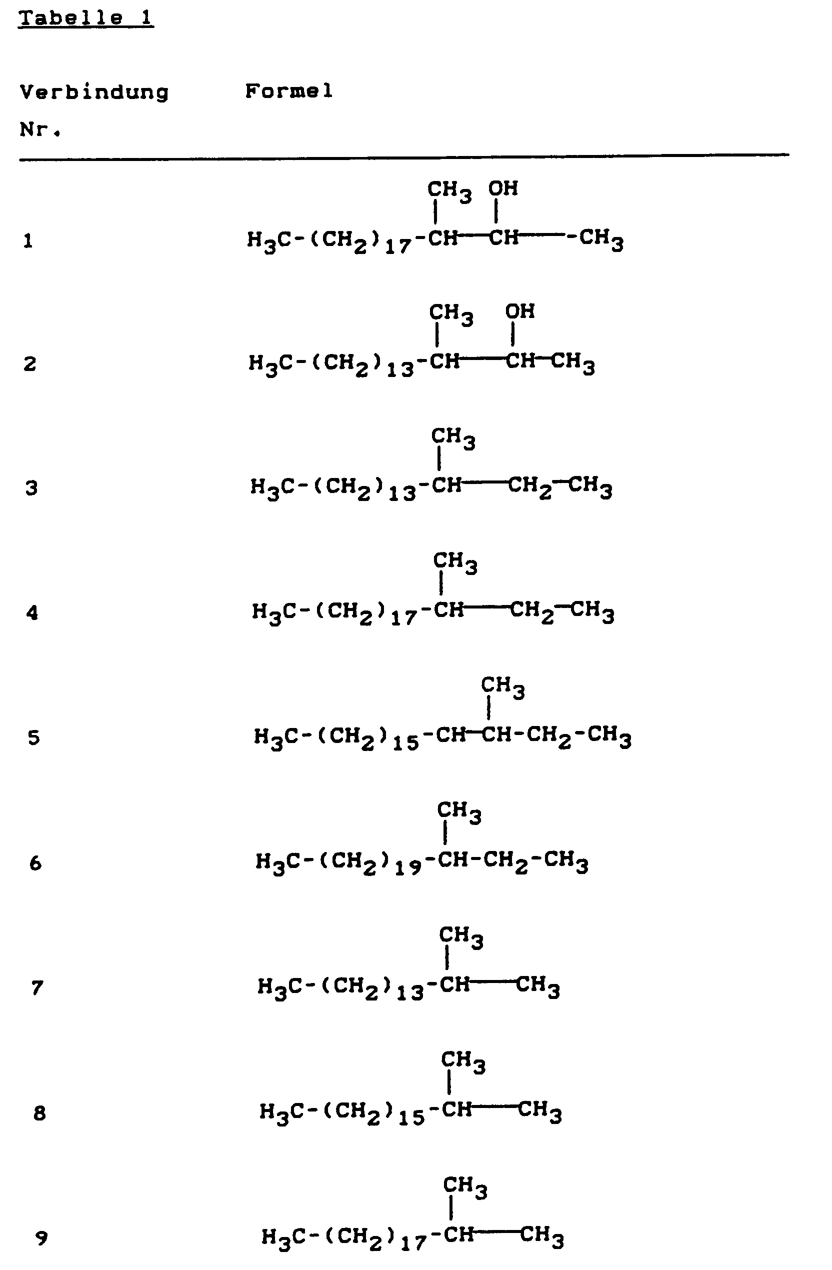

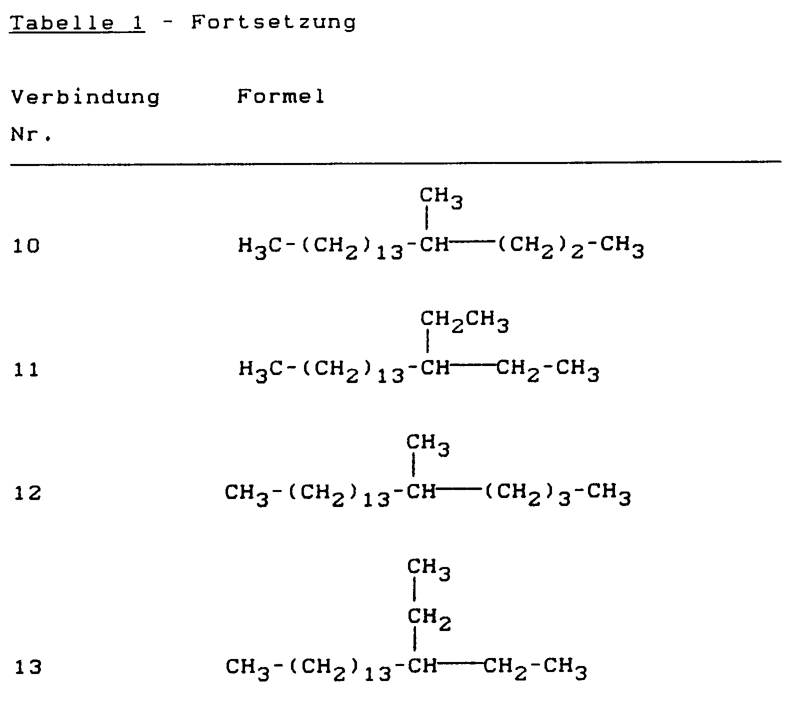

Die Verbindungen der allgemeinen Formel I sind teilweise bekannt (vgl. z.B. An. Bromatol. 30, 267 (1979); insect Biochem. 13, 381 (1983); J. Chem. Ecol. 6, 309 (1980); An. Bromatol. 31, 137 (1979); Biochem. Syst. Ecol. 16, 647 (1988); Riv. ital. Sostanze Grasse 63, 213 (1986); J. Chem. Ecol. 15, 939 (1989)) und können nach bekannten Verfahren und Methoden erhalten werden (vgl. z.B. Org. Reactions 4, 378 (1948); Chem. Rev. 65, 63 (1965). Viele der Verbindungen der allgemeinen Formel I können auch nach dem unten beschriebenen Verfahren hergestellt werden.

Die Verbindungen der allgemeinen Formel I, in welcher
- R¹: für Hydroxy-C₁-C₅-alkyl (vorzugsweise α-Hydroxy-C₁-C₅-alkyl) steht;
- R²: für C₁-C₅-Alkyl steht; und
- m: für eine ganze Zahl von 10 bis 20 steht,
sind neu und Teil der vorliegenden Erfindung, ausgenommen 3-Methyl-2-pentadecanol, bekannt aus Tetrahedron 48, 15. 3143 (1992) und 2- und 3-methyl-1-pentadecanol, bekannt aus Chemical Abs. 10^{TH} und 12^{TH} Coll. Chem. Substance Index. Für diese neuen Verbindungen gelten die oben erläuterten in Vorzugsbereichen aufgeführten Restedefinitionen und Restekombinationen als bevorzugte Definitionen und Restekombinationen.

In den ganz besonders bevorzugten neuen Verbindungen stehen
- R¹: für -CHOH-CH₃;
- R²: für Methyl und
- m: für eine ganze Zahl von 13 bis 17.

Die Herstellung der neuen Verbindungen der allgemeinen Formel I kann nach dem folgenden Verfahren erfolgen, das ebenfalls Teil der vorliegenden Erfindung ist. Man erhält die neuen Verbindungen, indem man

### Ketone der allgemeinen Formel II

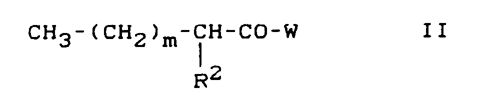

in welcher
- R² und m: die oben angegebene Bedeutung haben und
- W: für C₁-C₄-Alkyl steht,
mit einem Reduktionsmittel in Gegenwart eines Verdünnungsmittels umsetzt.

Verwendet man als Ausgangsstoff bei dem erfindungsgemäßen Verfahren beispielsweise 3-Methyl-heptadecan-2-on, so kann der Reaktionsverlauf durch folgendes Formelschema erläutert werden:

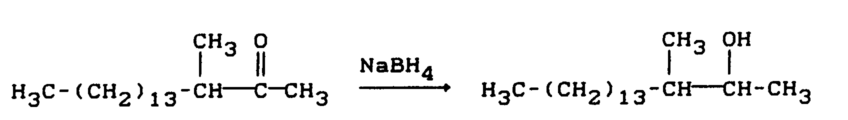

Die beim erfindungsgemäßen Verfahren als Ausgangsstoffe zu verwendenden Ketone der allgemeinen Formel II sind bekannt oder können nach bekannten Verfahren und Methoden hergestellt werden (vgl. Agr. Biol. Chem. 40, 391 (1976); J. Org. Chem. 40, 3456 (1975); J. Org. Chem. 40, 2410 (1975)).

Die oben aufgeführten allgemeinen oder in Vorzugsbereichen aufgeführten Restedefinitionen bzw. Erläuterungen können untereinander, also auch zwischen den jeweiligen Vorzugsbereichen beliebig kombiniert werden. Sie gelten für die Endprodukte sowie für die Vor- und Zwischenprodukte entsprechend.

Das erfindungsgemäße Verfahren wird vorzugsweise in Gegenwart eines Verdünnungsmittels durchgeführt. Als solche werden vorzugsweise polare Lösungsmittel wie Ethanol, Propanol, Dioxan, Dimethylsulfoxid und Dimethylformamid und ihre Mischungen verwendet. Niedere Alkylalkohole wie Ethanol und Propanol werden besonders bevorzugt.

Bei dem erfindungsgemäßen Verfahren werden Reduktionsmittel eingesetzt. Beispiele hierfür sind Lithiumborhydrid, Natriumborhydrid und Lithiumalanat sowie Wasserstoff in Gegenwart übliche Hydrierungskatalysatoren, wie PtO₂ oder Raney-Nickel. Besonders bevorzugt ist Natriumborhydrid.

Die Reaktionstemperaturen können bei dem erfindungsgemäßen Verfahren in einem größeren Bereich variiert werden. Vorzugsweise arbeitet man bei Temperaturen zwischen -20°C und +80°C, besonders bevorzugt bei Temperaturen zwischen 0°C und 40°C.

Zur Durchführung des erfindungsgemäßen Verfahrens setzt man je Mol Ausgangsverbindung der Formel II vorzugsweise zwischen 1 und 3 Mol, besonders bevorzugt 1 Mol Reduktionsmittel ein. Die Reaktionskomponenten werden vorzugsweise bei etwa 0°C zusammengegeben und bei etwas erhöhter Temperatur bis zum Ende der Umsetzung gerührt.

Die Aufarbeitung kann nach üblichen Methoden durchgeführt werden; beispielsweise indem man das Reaktionsgemisch einengt, den Rückstand in Wasser und Cyclohexan aufnimmt, mit Cyclohexan extrahiert, wiederum einengt und das Rohprodukt säulenchromatographisch reinigt.

Die Verbindungen der allgemeinen Formel I (im besonderen Maße die neuen Verbindungen) können erfindungsgemäß bei der Bekämpfung von Schaben, also Insekten der Ordnung Blattodea, insbesondere der Familie Blattellidae, vorzugsweise der Art Blattella germanica oder der Familie Blattidae, vorzugsweise der Arten Blatta orientalis und Periplaneta americana, aber auch gegen andere Schabenarten, ganz besonders bevorzugt jedoch gegen Blattella germanica, eingesetzt werden.

Die Verbindungen der allgemeinen Formel I beeinflussen dabei das Verhalten der Schaben derart, daß diese sich verstärkt an solchen Orten aufhalten oder solche Orte aufsuchen, die mit Verbindungen der allgemeinen Formel I behandelt wurden bzw. solche Verbindungen enthalten. Die Verbindungen der allgemeinen Formel I weisen somit starke anlockende Wirkungen auf. Die Verbindungen der Formel I können somit ganz allgemein bei der Schabenbekämpfung, unabhängig von der Art der angewendeten Bekämpfungsmethode eingesetzt werden. Sie können bei mechanischen Bekämpfungsverfahren beispielsweise und vorzugsweise in Schabenfallen oder Klebevorrichtungen oder bei biologischen oder chemischen Bekämpfungsverfahren oder bei einer Kombination solcher Bekämpfungsverfahren eingesetzt werden. Bei mechanischen Vorrichtungen können die Verbindungen der Formel I ganz oder weitflächig oder an geeigneten diskreten Stellen, z.B. durch Bestreichen, Aufsprühen, Imprägnieren, Aufdrucken, gegebenenfalls zusammen mit weiteren wirksamen Mitteln, wie anlockenden Farben, Ködermaterialien oder anderen Lockmitteln, Insektiziden usw. angebracht werden. Bei biologischen oder chemischen Bekämpfungsverfahren können die Verbindungen der Formel I mit den insektenpathogen wirkenden Organismen (z.B. Viren oder Mikroorganismen) oder den natürlichen oder synthetischen Insektiziden vermischt oder in ausreichender Nähe zu diesen Organismen oder Stoffen vorliegen. Bei einer Kombination mehrerer Bekämpfungsmethoden gilt entsprechendes.

Die Verbindungen der allgemeinen Formel I können als Einzelverbindungen oder in Mischung untereinander verwendet werden.

Dem Fachmann ist es anhand einfacher Überlegungen oder einfacher Untersuchungen leicht möglich, die für die jeweiligen Verwendungszwecke günstigen Verbindungen, Anwendungsarten und Mengen zu ermitteln.

Vorzugsweise werden die Verbindungen der allgemeinen Formel I in einen Insektizide enthaltenden Köder oder in Klebefallen eingearbeitet oder in der Nähe des Ködermaterials (z.B. darüber) angebracht. Die Verbindungen der allgemeinen Formel I können auch in einer Form vorliegen, in der sie über einen längeren Zeitraum freigegeben werden (Slow-release-Formulierungen). Hierzu können sie z.B. in Polymermaterial, Paraffinen, Wachsen usw. eingearbeitet werden oder mikroverkapselt vorliegen. Als Fallen können die üblichen Vorrichtungen dienen und als Ködermaterialien können übliche fraßattraktive Mittel dienen. Vorzugsweise werden die Verbindungen der allgemeinen Formel I in Mengen von 0,0001 bis 20 mg (ganz besonders bevorzugt 0,01 bis 0,1 mg) je Köder oder Falle, z.B. Klebefalle eingesetzt.

Die Verbindungen können auch in Form der üblichen Spritzmittel gegebenenfalls in Mischung mit geeigneten Insektiziden ausgebracht werden. Hierbei können die üblichen Formulierungen (z.B. auch Mikroverkapselung) verwendet werden, die mit den üblicherweise gebräuchlichen Applikationsgeräten ausgebracht werden können. Ebenso ist es möglich, die Verbindungen der allgemeinen Formel I, gegebenenfalls in Mischung mit geeigneten Insektiziden, zu streufähigen Stäuben oder Granulaten zu formulieren. Die Aufwandmengen an Verbindungen der allgemeinen Formel I liegen vorzugsweise bei 1 bis 500 mg je m² und besonders bevorzugt bei 2 bis 200 mg je m². Durch die Anwendung der Verbindungen der allgemeinen Formel I kann die Wirksamkeit von mechanischen Vorrichtungen (wie Fallen, Klebebänder) sowie insbesondere auch von insektiziden Mitteln erheblich verbessert werden. Teil der vorliegenden Erfindung sind somit auch mechanische Schabenbekämpfungsvorrichtungen und Mittel, die wenigstens eine Verbindung der allgemeinen Formel I enthalten. Bevorzugte mechanische Vorrichtungen und Mittel sind die üblichen Schabenfallen, die gegebenenfalls Köder, weitere Lock-oder Fraßstoffe und/oder insektizide Stoffe enthalten sowie Mittel und Fallen mit einer klebrigen Oberfläche an denen die Schaben haften bleiben, die gegebenenfalls neben üblichen Träger- und Hilfsstoffen Fraß- und Lockstoffe und/oder insektizid wirksame Stoffe wenigstens einer Verbindung der allgemeinen Formel I aufweisen. Hierbei können die Verbindungen der allgemeinen Formel I in der klebrigen Oberfläche enthalten sein oder in deren unmittelbaren Nähe angebracht vorliegen. In den mechanischen Vorrichtungen können auch Kombinationen von Fallen und Mitteln mit klebriger Oberfläche eingesetzt werden.

Ebenfalls Teil der vorliegenden Erfindung sind Schabenbekämpfungsmittel, welche gegebenenfalls neben üblichen Träger- und Hilfsstoffen und/oder sonstigen Zusatzstoffen (wie Ködermittel, Lockstoffe) wenigstens eine Verbindung der allgemeinen Formel I und wenigstens einen insektizid wirksamen Stoff enthalten, wobei die Verbindungen der Formel I mit den übrigen Bestandteilen vermischt sein können oder in einer separaten Anordnung vorliegen können. Diese Mittel können zusätzlich oder statt der insektizid wirksamen Stoffe auch entomopathogene Viren oder Mikroorganismen, die gegen Schaben wirksam sind, enthalten.

Als insektizide Stoffe können alle Stoffe verwendet werden, welche gegen Schaben wirksam sind, da es zu keiner unerwünschten Wechselwirkung zwischen den insektizid wirkenden Stoffen und den Verbindungen der allgemeinen Formel I kommt.

Insektizid wirkende Stoffe können beispielsweise den insektizid wirkenden Phosphorsäureestern, Carbamaten, natürlichen und synthetischen Pyrethroiden, Nitroimino-, Nitromethylen-, Cyanoimino- oder Cyanomethylen-Verbindungen, Pyrrolidin-2,4-dion-Derivaten und/oder Pyrazolin-Derivaten angehören.

Als erfindungsgemäß besonders bevorzugte insektizide Stoffe seien aufgeführt:

### 1) Carbamidsäureestern der Formel III

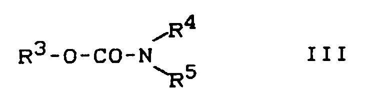

in welcher
- R³: für einen gegebenenfalls substituierten carbocyclischen oder heterocyclischen aromatischen Rest oder für einen gegebenenfalls substituierten Oximrest steht (wobei die weiter unten erläuterten Reste R³ bevorzugt werden),
- R⁴: für C₁-C₄-Alkyl steht und
- R⁵: für Wasserstoff, C₁-C₄-Alkyl oder für einen Rest u steht, wobei
- u: für den Rest -CO-R⁶ steht, worin
- R⁶: für Halogen, C₁-C₄-Alkyl, C₁-C₄-Alkoxy, C₃-C₅-Alkenoxy, C₃-C₅-Alkinoxy, C₁-C₄-Alkylthio, C₁-C₄-Alkyl-amino, Di-C₁-C₄-Alkylamino, C₁-C₄-Alkyl-hydroxylamino,
für gegebenenfalls durch Halogen, Nitro, Cyano, Trifluormethyl, C₁-C₄-Alkyl, C₁-C₄-Alkoxy, C₁-C₄-Alkylendioxy, C₁-C₄-Alkylthio, C₁-C₄-Alkoxy-carbonyl substituiertes Phenoxy, Phenylthio oder Phenylamino, für 2,3-Dihydro-2,2-dimethyl-7-benzofuranyl oder für den Rest 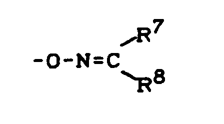 steht, worin
- R⁷: für Wasserstoff, C₁-C₄-Alkyl oder Di-C₁-C₄-alkyl-amino-carbonyl steht und
- R⁸: für C₁-C₄-Alkyl, C₁-C₄-Alkylthio, Cyano-C₁-C₄-alkylthio, C₁-C₄-Alkylthio-C₁-C₄-alkyl steht,
oder die beiden Reste R⁷ und R⁸ zusammen für gegebenenfalls durch Sauerstoff, Schwefel, SO oder SO₂ unterbrochenes C₂-C₈-Alkandiyl stehen, oder
in welcher
- u: für den Rest -S_{q}(O)ᵣ-R⁹ steht, worin
- q: für 1 oder 2 und
- r: für 0, 1 oder 2 stehen, wobei im Falle daß q für 2 steht, r 0 bedeutet und
- R⁹: für gegebenenfalls durch Halogen substituiertes C₁-C₄-Alkyl, C₃-C₅-Alkenyl, C₃-C₅-Alkinyl oder C₃-C₆-Cycloalkyl, für gegebenenfalls durch Halogen, Cyano, Nitro, Trifluormethyl, Trifluormethoxy, Trifluormethylthio, C₁-C₄-Alkyl oder C₁-C₄-Alkoxy substituiertes Phenyl, Benzyl oder Phenylethyl oder für den Rest 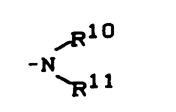 steht, worin
- R¹⁰: für C₁-C₄-Alkyl, C₃-C₅-Alkenyl, C₃-C₅-Alkinyl, C₃-C₆-Cycloalkyl oder Benzyl steht und
- R¹¹: für C₁-C₄-Alkyl, C₃-C₅-Alkenyl, C₃-C₅-Alkinyl, C₃-C₆-Cycloalkyl, Benzyl, Phenylethyl, Halogencarbonyl, Formyl, C₁-C₄-Alkyl-carbonyl, C₁-C₄-Alkoxy-carbonyl, C₁-C₄-Alkoxyphenoxy-carbonyl, C₃-C₅-Alkinoxy-carbonyl, C₃-C₅-Alkenoxycarbonyl, C₁-C₄-Alkylthiocarbonyl, C₁-C₄-Alkyl-amino-carbonyl, C₁-C₄-Alkyl-hyroxylamino-carbonyl, C₁-C₁₀-Alkyl-phenoxycarbonyl, Di-C₁-C₄-alkyl-aminocarbonyl, Phenylthiocarbonyl, Phenoxycarbonyl, 2,3-Dihydro-2,2-dimethyl-7-benzofuranyloxycarbonyl, für gegebenenfalls durch Halogen, Cyano, Nitro, Trifluormethyl, C₁-C₁₀-Alkyl oder C₁-C₄-Alkoxy substituietes Phenylsulfenyl, Phenylsulfinyl, Phenylsulfonyl oder Phenyl steht, oder für den Rest 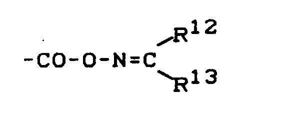
steht, worin
- R¹²: die oben für R⁷ angegebene Bedeutung und
- R¹³: die oben für R⁸ angegebene Bedeutung hat,
wobei ferner im Rest 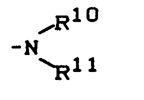 die Reste R¹⁰ und R¹¹ zusammen für eine gegebenenfalls durch Sauerstoff oder Schwefel unterbrochene Kohlenwasserstoffkette mit 3 bis 8 Kohlenstoffatomen stehen und worin weiter R⁹ auch für den gleichen Rest stehen kann, an den der Rest -S_{q}(O)ᵣ-R⁹ gebunden ist.

Als Wirkstoffkomponenten ganz besonders bevorzugt sind Carbamidsäureester der Formel III, in welcher
- R³: für gegebenenfalls durch C₁-C₄-Alkyl, C₂-C₄-Alkenyl, C₁-C₄-Alkoxy, C₁-C₄-Alkoxy-methyl, C₁-C₄-Alkylthio, C₁-C₄-Alkylthio-methyl, C₁-C₄-Alkylamino, Di-(C₁-C₄-alkyl)-amino, Di-(C₃-C₄-alkenyl)-amino, Halogen, Dioxolanyl, Methylendioxy und/oder durch den Rest -N=CH-N(CH₃)₂ substituierte Reste aus der Reihe Phenyl, Naphthyl, 2,3-Dihydro-7-benzofuranyl, Pyrazolyl oder Pyrimidinyl steht, oder in welcher
- R³: für einen Alkylidenaminorest der Formel 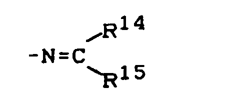
steht, in welcher
- R¹⁴ und R¹⁵: die oben für R⁷ bzw. R⁸ angegebene Bedeutung haben,
- R⁴: für C₁-C₄-Alkyl steht und
- R⁵: für Wasserstoff oder C₁-C₄-Alkyl (vorzugsweise für Wasserstoff) steht.

Als Beispiele für die Carbamidsäureester der Formel III seien die folgenden N-Methylcarbamidsäureester genannt: 2-Methyl-phenyl-, 2-Ethyl-phenyl-, 2-iso-Propyl-phenyl-, 2-sec.-Butyl-phenyl-, 2-Methoxy-phenyl-, 2-Ethoxy-phenyl, 2-iso-Propoxy-phenyl-, 4-Methyl-phenyl-, 4-Ethyl-phenyl-, 4-n-Propyl-phenyl-, 4-Methoxy-phenyl-, 4-Ethoxy-phenyl-, 4-n-Propoxy-phenyl-, 3,4,5-Trimethyl-phenyl-, 3,5-Dimethyl-4-methylthio-phenyl-, 3-Methyl-4-dimethylamino-phenyl-, 2-Ethylthiomethyl-phenyl-, 1-Naphthyl-, 2,3-Dihdyro-2,2-dimethyl-7-benzofuranyl-, 2,3-(Dimethylmethylendioxy)-phenyl-, 2-(4,5-Dimethyl-1,3-dioxolan-1-yl)-phenyl-, 1-Methylthio-ethyliden-amino-, 2-Methylthio-2-methylpropylidenamino-, 1-(2-Cyano-ethylthio)-ethylidenamino- und 1-Methylthiomethyl-2,2-dimethylpropylidenamino-N-methyl-carbamidsäureester, wobei das 2-iso-Propoxy-phenyl-N-methylcarbamat bevorzugt wird.

### 2) Carbonsäureester der Formel IV

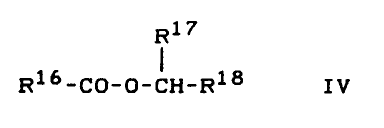

in welcher
- R¹⁶: für einen offenkettigen oder cyclischen Alkylrest steht, der gegebenenfalls substituiert ist durch Halogen, Alkyl, Cycloalkyl, durch gegebenenfalls durch Halogen, Alkyl und/oder Alkoxy substituiertes Alkenyl, durch Phenyl oder Styryl, welche gegebenenfalls durch Halogen, gegebenenfalls Halogensubstituierte Reste aus der Reihe Alkyl, Alkoxy, Alkylendioxy und/oder Alkylthio substituiert sind, durch spirocyclisch verknüpftes, gegebenenfalls Halogen-substituiertes Cycloalk(en)yl, welches gegebenenfalls benzannelliert ist, in welcher weiter
- R¹⁷: für Wasserstoff, Alkyl, Halogenalkyl, Alkenyl, Alkinyl oder Cyano steht, und
- R¹⁸: für einen gegebenenfalls substituierten Alkyl- oder Arylrest oder für einen Heterocyclus steht, oder zusammen mit R¹⁷ und dem Kohlenstoffatom, an das beide Reste gebunden sind, einen Cyclopentenonring bildet.

Ganz besonders als Wirkstoffkomponenten bevorzugt sind Carbonsäureester der Formel IV, in welcher
- R¹⁶: (a) für den Rest 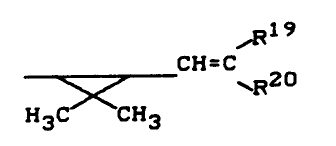
steht, worin
- R¹⁹: für Wasserstoff, Methyl, Fluor, Chlor oder Brom und
- R²⁰: für Methyl, Fluor, Chlor, Brom, C₁-C₂-Fluoralkyl oder C₁-C₂-Chlorfluoralkyl oder für gegebenenfalls durch Halogen und/oder gegebenenfalls Halogen-substituierte Reste der Reihe C₁-C₄-Alkyl, C₁-C₄-Alkoxy, C₁-C₄-Alkylthio und/oder C₁-C₂-Alkylendioxy substituiertes Phenyl steht, oder worin beide Reste R²⁵ und R²⁶ für C₂-C₅-Alkandiyl (Alkylen) stehen;
oder in welcher
- R¹⁶: (b) für den Rest 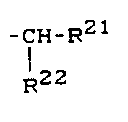 steht, worin
- R²¹: für gegebenenfalls durch Halogen und/oder durch gegebenenfalls Halogen-substituierte Reste der Reihe C₁-C₄-Alkyl, C₁-C₄-Alkoxy, C₁-C₄-Alkylthio oder C₁-C₂-Alkylendioxy substituiertes Phenyl steht und
- R²²: für Isopropyl oder Cyclopropyl steht;
oder in welcher
- R¹⁶: (c) für Methyl oder einen der Reste 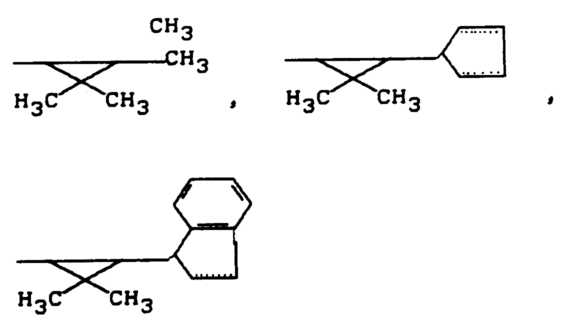 wobei die gepunkteten Linien mögliche Doppelbindungen andeuten sollen, steht,
und in welcher
- R¹⁶: für Wasserstoff, C₁-C₄-Alkyl, C₁-C₄-Halogenalkyl, Cyano oder Ethinyl steht und
- R¹⁸: für die Reste der Reihe Phenyl, Furyl oder Tetrahydrophthalimido steht, wobei diese Reste substituiert sein können durch Halogen und/oder Reste der Reihe C₁-C₄-Alkyl, C₂-C₄-Alkenyl, C₁-C₄-Alkoxy, C₂-C₄-Alkenoxy, C₁-C₄-Alkylthio, C₁-C₂-Alkylendioxy, Phenoxy und/oder Benzyl, welche ihrerseits durch Halogen substituiert sein können und wobei R¹⁸ vorzugsweise für Tetrafluorphenyl, 3,4-Dichlorphenyl, Tetrahydrophthalimido oder für Phenoxyphenyl, steht, welches in einem oder beiden Phenylringen durch Halogen (vorzugsweise Fluor) substituiert sein kann.

Weiter sind die natürlich vorkommenden Pyrethroide (wie Pyrethrum) als Carbonsäureester der Formel IV besonders bevorzugt.

Als Beispiele für erfindungsgemäß besonders bevorzugte Carbonsäureester der Formel IV seinen genannt
2,2-Dimethyl-3-(2-methyl-propen-1-yl)-cyclopropan-carbonsäure-(3,4,5,6-tetrahydro-phthalimido-methyl)-ester, 2,2-Dimethyl-3-(2,2-dichlorvinyl)-cyclopropan-carbonsäure-(3-phenoxy-benzyl)-ester, 2,2-Dimethyl-3-(2,2-dichlorvinyl)-cyclopropancarbonsäure-(α-cyano-3-phenoxy-benzyl)-ester, 2,2-Dimethyl-3-(2,2-dichlorvinyl)-cyclopropan-carbonsäure-(α-cyano-4-fluor-3-phenoxy-benzyl)-ester, 2,2-Dimethyl-3-(2,2-dichlorvinyl)-cyclopropancarbonsäure-(2,3,5,6-tetrafluor-benzyl)-ester, 2,2-Dimethyl-3-(2,2-dibromvinyl)-cyclopropancarbonsäure-(α-cyano-3-phenoxy-benzyl)-ester und 3-Methyl-2-(4-chlor-phenyl)-butansäure-(α-cyano-3-phenoxy-benzyl)-ester.

### 3) Phosphorsäure- und Phosphonsäureestern der allgemeinen Formel V

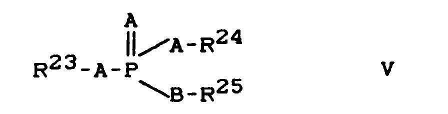

in welcher
- A: gleich oder verschieden ist und für O oder S steht und
- B: für O, S, -NH- oder für eine direkte Bindung zwischen dem zentralen P-Atom und R²⁵ steht und
- R²³ und R²⁴: gleich oder verschieden sind und für gegebenenfalls substituiertes Alkyl oder Aryl stehen,
- R²⁵: für Wasserstoff, gegebenenfalls substituiertes Alkyl, Aryl, Heteroaryl, Aralkyl, Alkenyl, Dioxanyl oder einen Oximrest oder für den gleichen Rest steht, an den es gebunden ist.

Besonders bevorzugt sind Phosphorsäure- und Phosphonsäureester der Formel V, in welcher
- R²³ und R²⁴: gleich oder verschieden sind und für C₁-C₄-Alkyl oder Phenyl stehen,
- R²⁵: für Wasserstoff, Alkyl mit 1 bis 4 Kohlenstoffatomen steht, das gegebenenfalls durch Halogen, Hydroxyl, Cyano, gegebenenfalls Halogen-substituiertes Phenyl, Carbamoyl, Alkylsulfonyl, Alkylsulfinyl, Alkylcarbonyl, Alkoxy, Alkylthio, Alkoxycarbonyl, Alkylaminocarbonyl, letztere mit jeweils bis zu 6 Kohlenstoffatomen, substituiert ist, für Alkenyl mit bis zu 4 Kohlenstoffatomen, das gegebenenfalls durch Halogen-substituiertes Phenyl oder C₁-C₄-Alkoxycarbonyl substituiert ist, oder für den Rest der allgemeinen Formel 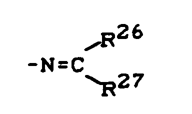 wobei R²⁶ und R²⁷ die oben für R⁷ bzw. R⁸ angegebene Bedeutung besitzen, oder für Cyano oder Phenyl stehen, und in welcher
- R²⁵: ferner für Dioxanyl, das durch denselben Rest substituiert ist, an den R²⁴ gebunden ist, oder R²⁵ für den gleichen Rest an den es gebunden ist steht, oder R²⁵ für Phenyl, das gegebenenfalls durch Methyl, Nitro, Cyano, Halogen und/oder Methylthio substituiert ist steht, wobei
- R²⁵: außerdem besonders bevorzugt für gegebenenfalls durch C₁-C₄-Alkoxy, C₁-C₄-Alkylthiomethyl, C₁-C₄-Alkyl und/oder durch Halogen substituierte heteroaromatische Reste, wie Pyridinyl, Chinolinyl, Chinoxalinyl, Pyrimidinyl oder Benzo-1,2,4-triazinyl steht.

Im einzelnen seien genannt:
O,O-Dimethyl- bzw. O,O-Diethyl-O-(2,2-dichlor- bzw. 2,2-dibromvinyl)-phosphorsäureester,
O,O-Diethyl-O-(4-nitro-phenyl)-thionophosphorsäureester,
O,O-Dimethyl-O-(3-methyl-4-methylthio-phenyl)-thionophosphorsäureester,
O,O-Dimethyl-O-(3-methyl-4-nitro-phenyl)-thionophosphorsäureester,
O-Ethyl-S-n-propyl-O-(2,4-dichlorphenyl)-thionophosphorsäureester,
O-Ethyl-S-n-propyl-O-(4-methylthio-phenyl)-thionophosphorsäureester,
O,O-Dimethyl-S-(4-oxo-1,2,3-benzotriazin(3)yl-methyl)-thionothiolphosphorsäureester,
O-methyl-O-(2-iso-propyl-6-methoxy-pyrimidin(4)yl)-thionomethanphosphonsäureester,
O,O-Diethyl-O-(2-iso-propyl-6-methyl-pyrimidin(4)yl)-thionophosphorsäureester,
O,O-Diethyl-O-(3-chlor-4-methyl-cumarin(7)yl)-thionophosphorsäureester,
O,O-Dimethyl-2,2,2-trichlor-1-hydroxy-ethan-phosphonsäureester,
O,O-Dimethyl-S-(methylaminocarbonyl-methyl)-thionophosphonsäureester,
O-Methyl-O-(6-methoxy-2-tert.-butyl-pyrimidin-4-yl)-thionoethan-phosphonsäure-diester.

### 4) Nitromethylen-, Nitroimino-, Cyanoimino- oder Cyanomethylen-Derivate der Formel VI

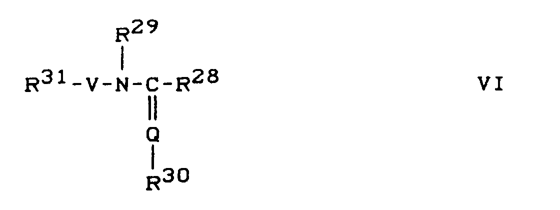

in welcher
- R²⁸: für C₁-C₄-Alkyl (vorzugsweise für Methyl oder Ethyl) oder für die Gruppe 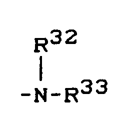 steht, in welcher
- R³²: C₁-C₄-Alkyl (vorzugsweise Methyl oder Ethyl) bedeutet oder
- R³²: gemeinsam mit R²⁹ eine gegebenenfalls verzweigte C₂-C₅-Alkandiyl-Kette (vorzugsweise -(CH₂)₂- oder -(CH₂)₃-) darstellt,
und
- R³³: Wasserstoff oder C₁-C₄-Alkyl (vorzugsweise Wasserstoff) bedeutet;
- R²⁹: für C₁-C₄-Alkyl (vorzugsweise Methyl oder Ethyl) steht oder gemeinsam mit R³² eine gegebenenfalls verzweigte C₂-C₅-Alkandiyl-Kette (vorzugsweise -(CH₂)₂- oder -(CH₂)₃-) darstellt,
- R³⁰: für NO₂ oder CN steht;
- R³¹: für einen gegebenenfalls substituierten (vorzugsweise durch Halogen und/oder C₁-C₄-Alkyl substituierten) heteroaromatischen Rest (vorzugsweise den Pyridylrest) steht (wobei R³¹ besonders bevorzugt für die 2-Chlorpyridyl-5-Gruppe steht);
- Q: für =C- oder =N- steht; und
- V: für eine direkte Bindung oder für einen C₁-C₃-Alkandiyl-Rest (vorzugsweise für -CH₂-) steht.

Im folgenden werden beispielhaft einige besonders bevorzugte Verbindungen der Formel VI speziell aufgeführt: 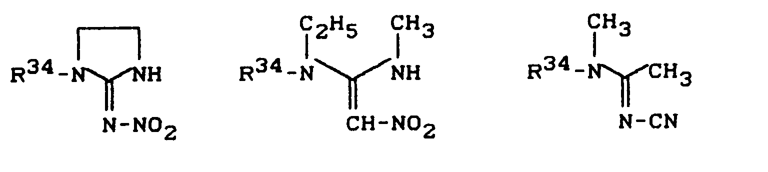 wobei R³⁴ den 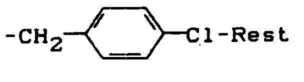 bedeutet.

Die Verbindungen der Formel I und/oder die insektiziden Stoffe sowie die Mischungen der Verbindungen der Formel I mit den insektiziden Stoffen können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Hilfs- und/oder Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, PolyoxyethylenFettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen können im Falle von Köderformulierungen (vorzugsweise Streuköderformulierungen oder Festköderformulierungen) zusätzlich weitere Zusatzstoffe enthalten, die die Schaben anlocken und/oder zur Aufnahme der insektiziden Stoffe veranlassen. Als Lockstoffe und Fraßstoffe können alle üblicherweise verwendeten Präparate verwendet werden, wie natürliche oder synthetische Geruchsstoffe, Farbstoffe und/oder Stoffe, die von Schaben gerne aufgenommen werden, wie stärkehaltige und/oder zuckerhaltige Produkte auf Getreidebasis.

Die Formulierungen enthalten vorzugsweise 0,001 bis 95, insbesondere 0,01 bis 70, Gewichtsprozente an Verbindungen der Formel I.

Die Formulierungen der insektiziden Stoffe enthalten (gegebenenfalls neben den Verbindungen der Formel I) vorzugsweise zwischen 0,1 und 95, insbesondere 0,5 und 90 Gewichtsprozente insektizide Stoffe.

Die Anwendung der Schabenbekämpfungsmittel erfolgt in einer an die Anwendungsform angepaßten üblichen Weise.

Die biologische Wirksamkeit der Verbindungen der Formel I soll anhand der folgenden Beispiele erläutert werden.

### A) Verbesserung der Wirksamkeit einer Köderformulierung

In einem Raum (2,25 x 4,70 m) befinden sich in zwei gegenüberliegenden Ecken je ein Versteck sowie je eine Tränke, in den anderen zwei Ecken je ein Stück Zwieback. Einen Tag nach Freilassen von 20 männlichen und 20 weiblichen Schaben (Blattella germancia) wird in 40 cm Wandabstand zum Zwieback je eine Köderdose mit einer insektizidhaltigen Fraßtablette (im vorliegenden Fall 0,5 % Ethylchlorpyrifos) aufgestellt.

Auf der Innenseite des Köderdosendeckels befindet sich ein Plättchen, das 1 Stunde zuvor mit einer Verbindung der Formel I (gelöst in n-Hexan) oder nur mit n-Hexan behandelt worden war (unbehandelter Köder = Kontrolle)
3 Räume wurden jeweils mit behandelten Köderdosen und 3 mit unbehandelten Köderdosen besetzt.

Am folgenden Tag wurde die Mortalität der männlichen und weiblichen Tiere bestimmt.

Die Versuchsergebnisse sind aus der folgenden Tabelle 2 ersichtlich:

**Tabelle 2**

| eingesetzte Verbindung der Tabelle 1 | Mortalität in % nach 1 Tag (Mittel aus 3 Versuchen) | |
|---|---|---|
| | männliche Schaben | weibliche Schaben |
| 1 Kontrolle | 47 | 48 |
| | 42 | 37 |
| 2 Kontrolle | 38 | 28 |
| | 27 | 25 |
| 3 Kontrolle | 78 | 62 |
| | 25 | 33 |
| 4 Kontrolle | 53 | 54 |
| | 33 | 29 |

### B) Verbesserung der Wirksamkeit eines Spritzmittels

Keramische Kacheln werden mit einer wäßrigen Spritzbrühe besprüht, die (a) eine 50 g/Liter enthaltende Cyfluthrin-Formulierung und (b) diese Formulierung und zusätzlich die Verbindung 3 aus Tabelle 1 enthält. Die Aufwandmenge an Cyfluthrin beträgt jeweils 20 mg Wirkstoff je m² Kachel. Die Aufwandmenge Verbindung Nr. 3 beträgt 4 mg Wirkstoff je m² Kachel.

Je eine dieser Kacheln wird in die Ecke eines Behälters (49 x 59 cm, Höhe 29,5 cm) gelegt, in dem sich eine Tränke, Futter, ein Versteck sowie 2 Stunden zuvor eingesetzte Männchen und Weibchen von Blattella germanica (je 5 Stück) befinden. Jeder Versuch besteht aus 3 Wiederholungen. Nach 1, 2 und 3 Tagen wird die Mortalität bestimmt und der Mittelwert aus den 3 Versuchen gebildet.

Der Versuch ergab folgende Ergebnisse:

| Kacheln behandelt mit (Wirkstoff/m²) | Mortalität in % nach | | |
|---|---|---|---|
| | 1 Tag | 2 Tagen | 3 Tagen |
| 20 mg Cyfluthrin + 4 mg Verbindung Nr. 3 aus Tabelle 1 | 70 | 90 | 93 |
| 20 mg Cyfluthrin (Kontrolle) | 43 | 70 | 70 |

Die Herstellung der Verbindungen der allgemeinen Formel I soll anhand der folgenden Beispiele erläutert werden:

### Beispiel 1

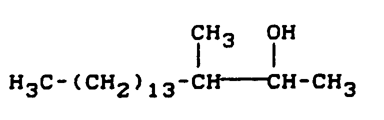

(Verbindung 2 aus Tabelle 1)
2 g (7,46 mmol) 3-Methyl-heptadecan-2-on werden in 20 ml Ethanol gelöst und bei 0°C mit 0,28 g (7,46 mmol) Natriumborhydrid versetzt. Zwei Stunden wird bei 0°C und zwei Stunden bei Raumtemperatur nachgerührt. Anschließend wird unter vermindertem Druck eingeengt, in Cyclohexan-Wasser aufgenommen und die wäßrige Phase dreimal mit Cyclohexan extrahiert. Nach Trocknen und Einengen fallen 2 g (99 % der Theorie) 3-Methyl-heptadecan-2-ol als reine Substanz an.

¹H-NMR (200 MHz, CDCl₃): δ = 0,9 (2d, 6H), 1,18 (t, 3H), 1,2-1,6 (m, 27H), 3,65 (m, 1H)
Analog wird die Verbindung Nr. 1 der Tabelle 1 synthetisiert:

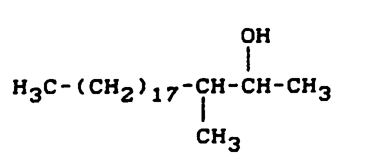

¹H-NMR (200 MHz, CDCl₃): δ = 0,88 (d + t, 6H), 1,15 (t, 3H), 1,20-1,40 (m, 35 H), 3,60-3,75 (m, 1H).

### Beispiel 2

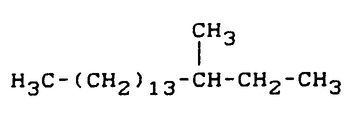

(Verbindung Nr. 3 aus Tabelle 1)
2 g (7,46 mmol) 3-Methyl-heptadecan-2-on (Verbindung Nr. 3 der Tabelle 1) werden in 10 ml Diethylenglykol vorgelegt, mit 1,11 g (22,4 mmol) Hydrazinhydrat und 1,2 g (29,85 mmol) Natriumhydroxid versetzt. Zwanzig Stunden wird bei 200°C gerührt. Nach Abkühlen auf Raumtemperatur wird mit 100 ml Wasser versetzt, unter Kühlung mit Salzsäure angesäuert und dreimal mit Cyclohexan extrahiert. Die vereinigten organischen Extrakte werden zweimal mit wenig Wasser gewaschen, über Natriumsulfat getrocknet und eingeengt. Die säulenchromatographische Trennung mit dem Laufmittel Cyclohexan liefert 1,0 g (53 % der Theorie) 3-Methylheptadecan.
- MS m/z (%):: 254 (23, M⁺), 225 (46), 196 (18), 155 (17), 113 (22), 99 (28), 85 (67), 71 (83), 57 (100).

Entsprechend dieser Vorschrift wird auch die Verbindung Nr. 4 der Tabelle 1 synthetisiert

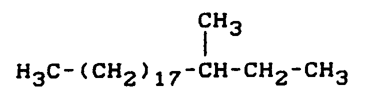

¹H-NMR (200 MHz, CCl₃): δ = 0,85 (t, 9H), 1,1-1,4 (m, 37H).

Die Synthese der Ausgangsverbindungen kann nach bekannten Herstellungsverfahren (vgl. Agr. Biol. Chem. 40, 391 (1976); J. Org. Chem. 42, 566 (1977) erfolgen.

## Patentansprüche

1. Verwendung von Verbindungen der allgemeinen Formel I 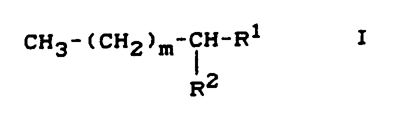 in welcher
R¹ für C₁-C₅-Alkyl oder Hydroxy-C₁-C₅-alkyl, steht;
R² für C₁-C₅-Alkyl steht und
m für eine ganze Zahl von 10 bis 20 steht,
bei der Bekämpfung von Schaben.

2. Verwendung gemäß Anspruch 1, wobei in der allgemeinen Formel I
R¹ für C₁-C₄-Alkyl oder Hydroxy-C₁-C₄-alkyl, steht,
R² für C₁-C₃-Alkyl steht; und
m für eine ganze Zahl von 11 bis 19 steht.

3. Verwendung gemäß Anspruch 1, wobei in der allgemeinen Formel I
R¹ für C₁-C₃-Alkyl oder Hydroxy-C₁-C₃-alkyl, steht;
R² für Methyl oder Ethyl steht; und
m für eine ganze Zahl von 12 bis 18 steht.

4. Verwendung gemäß Anspruch 1 bei der Schabenbekämpfung mit mechanischen Schabenbekämpfungsvorrichtungen und Mitteln, mit biologischen oder chemischen Mitteln und mit Kombinationen solcher Vorrichtungen und Mittel.

5. Mechanische Schabenbekämpfungsvorrichtungen und Mittel, welche wenigstens eine Verbindung der allgemeinen Formel I gemäß Anspruch 1 enthalten.

6. Schabenbekämpfungsmittel, welche
(a) wenigstens eine Verbindung der allgemeinen Formel I gemäß Anspruch 1 und
(b) wenigstens einen entomopathogenen Virus oder Mikroorganismus und/oder wenigstens einen insektizid wirkenden Stoff und gegebenenfalls
(c) ein oder mehrere Hilfs- und/oder Streckmittel und/oder sonstige Zusatzstoffe enthalten,
wobei die obigen Bestandteile in einer Mischung oder in einer separaten Anordnung vorliegen können.

7. Mechanische Schabenbekämpfungsvorrichtungen und Mittel nach Anspruch 5, welche ein Schabenbekämpfungsmittel gemäß Anspruch 6 enthalten.

8. Schabenbekämpfungsmittel, welche eine klebrige Oberfläche aufweisen, an denen die Schaben haften bleiben und welche wenigstens eine Verbindung der allgemeinen Formel I gemäß Anspruch 1 in der klebrigen Oberflächenschicht oder in unmittelbarer Nähe der klebrigen Oberfläche enthalten.

9. Verbindungen der allgemeinen Formel I 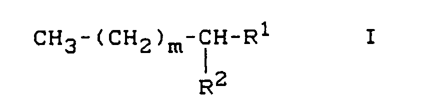 in welcher
R¹ für Hydroxy-C₁-C₅-alkyl steht;
R² für C₁-C₅-Alkyl steht; und
m für eine ganze Zahl von 10 bis 20 steht, ausgenommen 3-Methyl-2-pentadecanol 2-methyl-1-pentadecanol und 3-methyl-1-pentadecanol.

10. Verfahren zur Herstellung von Verbindungen der allgemeinen Formel I 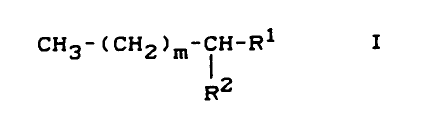 in welcher
R¹ für Hydroxy-C₁-C₅-Alkyl steht,
R² für C₁-C₅-Alkyl steht; und
m für eine ganze Zahl von 0 bis 15 steht,
dadurch gekennzeichnet, daß man
Ketone der allgemeinen Formel II 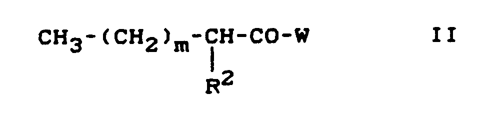 in welcher
R² und m die oben angegebene Bedeutung haben und
W für C₁-C₄-Alkyl steht,
mit einem Reduktionsmittel in Gegenwart eines Verdünnungsmittels umsetzt.

## Claims

1. Use of compounds of the general formula I 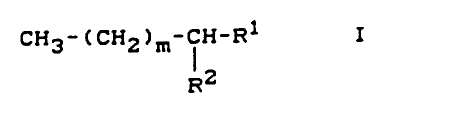 in which
R¹ represents C₁-C₅-alkyl or hydroxy-C₁-C₅-alkyl;
R² represents C₁-C₅-alkyl; and
m represents an integer from 10 to 20,
for combating cockroaches.

2. Use according to Claim 1, where, in the general formula I,
R¹ represents C₁-C₄-alkyl or hydroxy-C₁-C₄-alkyl,
R² represents C₁-C₃-alkyl; and
m represents an integer from 11 to 19.

3. Use according to Claim 1, where, in the general formula I,
R¹ represents C₁-C₃-alkyl or hydroxy-C₁-C₃-alkyl;
R² represents methyl or ethyl; and
m represents an integer from 12 to 18.

4. Use according to Claim 1 for combating cockroaches using mechanical devices and agents for combating cockroaches, biological or chemical agents and combinations of such devices and agents.

5. Mechanical devices for combating cockroaches and agents which contain at least one compound of the general formula I according to Claim 1.

6. Agents for combating cockroaches which contain
(a) at least one compound of the general formula I according to Claim 1 and
(b) at least one entomopathogenic virus or microorganism and/or at least one insecticidally active substance and, if appropriate,
(c) one or more auxiliaries and/or extenders and/or other additives,
it being possible for the above components to exist in the form of a mixture or as a separate arrangement.

7. Mechanical devices and agents for combating cockroaches according to Claim 5 which contain an agent for combating cockroaches according to Claim 6.

8. Agents for combating cockroaches which have a tacky surface to which the cockroaches adhere and which contain at least one compound of the general formula I according to Claim 1 in the tacky surface layer or in immediate vicinity of the tacky surface.

9. Compounds of the general formula I 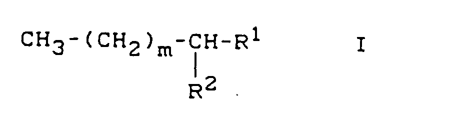 in which
R¹ represents hydroxy-C₁-C₅-alkyl;
R² represents C₁-C₅-alkyl; and
m represents an integer from 10 to 20, with the exception of 3-methyl-2-pentadecanol, 2-methyl-1-pentadecanol and 3-methyl-1-pentadecanol.

10. Process for the preparation of compounds of the general formula I 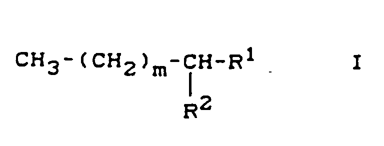 in which
R¹ represents hydroxy-C₁-C₅-alkyl,
R² represents C₁-C₅-alkyl; and
m represents an integer from 0 to 15,
characterised in that
ketones of the general formula II 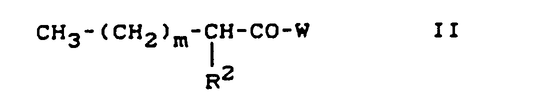 in which
R² and m have the abovementioned meaning and
W represents C₁-C₄-alkyl,
are reacted with a reducing agent in the presence of a diluent.

## Revendications

1. Utilisation de composés de formule générale I 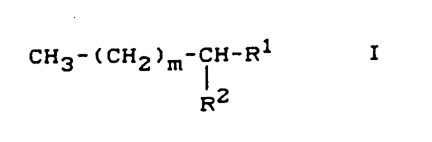 dans laquelle
R¹ est un groupe alkyle en C₁ à C₅ ou un groupe hydroxyalkyle en C₁ à C₅ ;
R² est un groupe alkyle en C₁ à C₅ et
m est un nombre entier de 10 à 20,
dans la lutte contre des blattes.

2. Utilisation suivant la revendication 1, dans laquelle
R¹ est un groupe alkyle en C₁ à C₄ ou un groupe hydroxyalkyle en C₁ à C₄,
R² est un groupe alkyle en C₁ à C₃ ; et
m est un nombre entier de 11 à 19,
dans la formule générale I.

3. Utilisation suivant la revendication 1, dans laquelle
R¹ est un groupe alkyle en C₁ à C₃ ou hydroxyalkyle en C₁ à C₃ ;
R² est un groupe méthyle ou éthyle ; et
m est un nombre entier de 12 à 18,
dans la formule générale I.

4. Utilisation suivant la revendication 1 dans la lutte contre des blattes avec des dispositifs et moyens mécaniques pour combattre les blattes, avec des moyens biologiques ou chimiques et avec des associations de ces dispositifs et moyens.

5. Dispositifs et moyens mécaniques pour combattre des blattes, qui contiennent au moins un composé de formule générale I suivant la revendication 1.

6. Compositions pour combattre des blattes, qui contiennent
(a) au moins un composé de formule générale I suivant la revendication 1 et
(b) au moins un virus ou un micro-organisme entomopathogène et/ou au moins une substance douée d'activité insecticide et, le cas échéant
(c) une ou plusieurs substances auxiliaires et/ou un ou plusieurs diluants et/ou d'autres additifs,
les constituants indiqués ci-dessus pouvant exister en mélange ou avoir une disposition séparée.

7. Dispositifs et moyens mécaniques pour combattre les blattes suivant la revendication 5, qui contiennent une composition pour combattre les blattes suivant la revendication 6.

8. Moyens de lutte contre les blattes, présentant une surface adhésive, auxquels les blattes restent collées et qui contiennent au moins un composé de formule générale I suivant la revendication 1 dans la couche superficielle adhésive ou au voisinage direct de la surface adhésive.

9. Composés de formule générale I 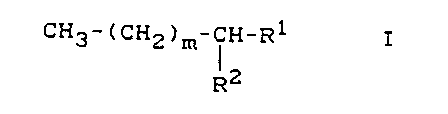 dans laquelle
R¹ est un groupe hydroxyalkyle en C₁ à C₅ ;
R² est un groupe alkyle en C₁ à C₅ ; et
m est un nombre entier de 10 à 20, à l'exclusion du 3-méthyl-2-pentadécanol, du 2-méthyl-1-pentadécanol et du 3-méthyl-1-pentadécanol.

10. Procédé de production de composés de formule générale I 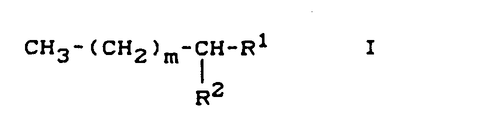 dans laquelle
R¹ est un groupe hydroxyalkyle en C₁ à C₅,
R² est un groupe alkyle en C₁ à C₅ ; et
m est un nombre entier de 0 à 15,
caractérisé en ce qu'on fait réagir avec un agent réducteur, en présence d'un diluant, des cétones de formule générale II 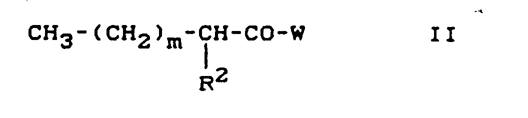 dans laquelle
R² et m ont les définitions indiquées ci-dessus et
W est un groupe alkyle en C₁ à C₄.
